# EUROPÄISCHE PATENTSCHRIFT

(11) **EP 0 583 672 B1**
(45) Veröffentlichungstag und Bekanntmachung des Hinweises auf die Patenterteilung: **13.03.1996**
(21) Anmeldenummer: 93112352.5
(22) Anmeldetag: 02.08.1993
(51) Int. Cl.: C07C 303/20

(54) **Verfahren zur Herstellung von 2-Aryl-ethan-sulfonsäuren**
Process for the preparation of 2-aryl-ethane-sulfonic acids
Procédé de préparation d'acides 2-aryl-éthane sulfoniques

(30) Priorität: 14.08.1992 DE 4227027
(43) Veröffentlichungstag der Anmeldung: 23.02.1994
(73) Patentinhaber: BAYER AG, D-51368 Leverkusen (DE)
(72) Erfinder: Meier, Jürgen-Dietrich, Dr., D-51375 Leverkusen (DE); Münster, Christian, D-51381 Leverkusen (DE); Käss, Volker, Dr., D-51375 Leverkusen (DE); Siffrin, Horst, Miles Inc., New Martins Ville, WV 26155-0500 (US)

(56) Entgegenhaltungen:
- DE-A- 1 643 585
- US-A- 3 479 397
- CHEMICAL ABSTRACTS, vol. 84, 1976, Columbus, Ohio, US; abstract no. 163811p,
- CHEMICAL ABSTRACTS, vol. 104, 1986, Columbus, Ohio, US; abstract no. 68760u, & JP-A-60 178 863

## Beschreibung

Die Erfindung betrifft ein Verfahren zur Herstellung von 2-Aryl-ethan-sulfonsäuren durch Umsetzung der zugrundeliegenden Vinylaromaten mit schwefliger Säure, das dadurch gekennzeichnet ist: daß man die Umsetzung in Gegenwart eines Amins vornimmt.

Die Titelverbindungen sind wertvolle Zwischenprodukte; beispielsweise sind 2-Heteroaryl-ethan-sulfonsäuren wie die 2-[4]-Pyridyl-ethan-sulfonsäure, wertvolle Zwischenprodukte zur Herstellung von Fotochemikalien und Pharmazeutika. Solche 2-Aryl-ethan-sulfonsäuren erhält man in bekannter Weise durch Umsetzen der zugrundeliegenden Vinylaromaten mit SO₂. Gemäß US 2.508.904 werden einige Pyridyl-ethan-sulfonsäuren durch Umsetzung der zugrundeliegenden Vinyl-pyridine mit SO₂ in einem alkoholischwäßrigen Medium erhalten. Anstelle von SO₂ kann auch NaHSO₃ eingesetzt werden. Da hierbei dunkel gefärbte Nebenprodukte entstehen, ist die so hergestellte Pyridyl-ethan-sulfonsäure etwa für Fotochemikalien ungeeignet; es ist in solchen Fällen eine aufwendige und mit Verlusten verbundene Umkristallisation erforderlich.

Gemäß JP 60/178 863 (1985) kann man auf die Mitbenutzung eines organischen Lösungsmittels verzichten und nur in Wasser als Reaktionsmedium arbeiten. Die Ausbeute ist jedoch mit 65,8 % nur mäßig.

Es wurde nun überraschend gefunden, daß 2-Aryl-ethan-sulfonsäuren in glatter Reaktion erhalten werden, wenn man die literaturbekannte Reaktion in Gegenwart eines weiter unten näher beschriebenen Amins durchführt.

Die vorliegende Erfindung betrifft ein Verfahren zur Herstellung von 2-Aryl-ethan-sulfonsäuren oder ihren Salzen, die in Form der freien Säuren der Formel

Ar¹-CH₂-CH₂-SO₃H (I)

entsprechen, in der
- Ar¹: einen carbocyclischen oder heterocyclischen aromatischen Rest, der mit einem anellierten Benzolkern verbunden sein kann, bedeutet,
durch Umsetzung von Vinylaromaten der Formel

Ar¹-CH=CH₂ (II),

in der
- Ar¹: die obige Bedeutung hat,
mit schwefliger Säure oder ihren löslichen Salzen oder ihrem Anhydrid in einem Wasser enthaltenden Medium, das dadurch gekennzeichnet ist, daß die Umsetzung bei einer Temperatur von -5°C bis +65°C und einem Druck von 0,5 bis 5 bar in Gegenwart eines Amins der Formel

N(R¹,R,R³) (III)

durchgeführt wird, in der
- R¹, R und R³: unabhängig voneinander geradkettiges oder verzweigtes C₁-C₁₂-Alkyl, C₃-C₈-Cycloalkyl, Phenyl oder substituiertes Phenyl oder C₇-C₁₀-Aralkyl bedeuten, wobei weiterhin 2 der Reste R¹, R und R³ gemeinsam mit dem N-Atom, das sie substituieren, einen 5- bis 7-gliedrigen, gesättigten heterocyclischen Ring bilden können, der ein oder zwei weitere Heteroatome aus der Gruppe N, O und S enthalten kann, wobei weiterhin bis zu zwei der Substituenten R¹, R und R³ Wasserstoff bedeuten können und wobei weiterhin alle drei Reste R¹, R und R³ gemeinsam mit dem N-Atom, das sie substituieren, ein bicyclisches tertiäres Amin oder das Pyridinsystem bilden können.

Ar¹ bedeutet einen carbocyclischen oder heterocyclischen aromatischen Rest, der mit anellierten Benzolkernen verbunden sein kann. Carbocyclische Reste dieser Art sind beispielsweise Phenyl, Naphthyl, Biphenylyl, bevorzugt Phenyl. Solche carbocyclischen aromatischen Reste können weiterhin durch C₁-C₄-Alkyl, wie Methyl, Ethyl, Propyl, Isopropyl, Butyl, Isobutyl, durch C₁-C₄-Alkoxy, wie Methoxy, Ethoxy, Propoxy, Isopropoxy, Butoxy oder Isobutoxy, mit Chlor, Brom oder Fluor ein- bis dreifach, bevorzugt einfach substituiert sein. Heterocyclische aromatische Reste sind Fünfringe oder Sechsringe mit einem oder mehreren Heteroatomen aus der Gruppe N, S und O, die weiterhin mit einem anellierten Benzolkern verbunden sein können, wie die Reste von Pyrrol, Furan, Thiophen, Indol, Thionaphthen, Pyrazol, Imidazol, Benzimidazol, Oxazol, Thiazol, Benzthiazol, Triazol, Pyridin, Chinolin, Isochinolin, Acridin, Pyridazin, Pyrimidin, Pyrazin, Thiazin, Oxazin, Chinoxalin, Triazin und andere dem Fachmann geläufige. Heterocyclische aromatische Reste der genannten Art können ihrerseits durch C₁-C₄-Alkyl, C₁-C₄-Alkoxy, Chlor, Brom oder Fluor substituiert sein.

In bevorzugter Weise tritt an die Stelle von Ar¹ der Rest Ar, der einen heterocyclischen aromatischen Rest bedeutet, der mit einem anellierten Benzolkern verbunden sein kann.

In besonders bevorzugter Weise tritt an die Stelle von Ar der Rest Ar³, der einen Pyridinrest bedeutet, der mit anellierten Benzolkernen verbunden sein kann. Auch Ar und Ar³ können in der unter Ar¹ beschriebenen Weise substituiert sein.

Dabei handelt es sich um Vinylaromaten der Formeln

Ar-CH=CH₂ (IV)

bzw.

Ar³-CH=CH₂ (V)

Geradkettiges oder verzweigtes C₁-C₁₂-Alkyl bedeutet außer dem genannten C₁-C₄-Alkyl noch eines der isomeren Pentyle, Hexyle, Octyle, Decyle oder Dodecyle.

C₃-C₈-Cycloalkyl bedeutet Cyclopropyl, Cyclobutyl, Cyclopentyl, Cyclohexyl, Cycloheptyl, Cyclooctyl, Methyl-oder Dimethyl-cyclopropyl, Methyl- oder Dimethyl-cyclopentyl, Methyl- oder Dimethyl-cyclohexyl oder Methyl-cycloheptyl.

Phenyl oder substituiertes Phenyl ist eines, wie es oben unter Ar¹ beschrieben wurde.

C₇-C₁₀-Aralkyl ist beispielsweise Benzyl, 1-Phenylethyl, 2-Phenyl-ethyl, Phenyl-propyl oder Phenyl-butyl, bevorzugt Benzyl, wobei der aromatische Teil in der weiter oben beschriebenen Weise substituiert sein kann.

Zwei der Reste R¹, R und R³ können gemeinsam mit dem N-Atom, das sie substituieren, einen 5- bis 7-gliedrigen, gesättigten heterocyclischen Ring bilden. Dieser kann ein oder zwei weitere Heteroatome aus der Gruppe N, O und S enthalten. Es handelt sich sodann um Abkömmlinge von cyclischen Aminen vom Typ des Pyrrolidins, des Oxazolidins, des Thiazolidins, des Piperidins, des Morpholins und anderer dem Fachmann bekannter Verbindungen dieser Art. Schließlich können alle drei Reste R¹, R und R³ gemeinsam mit dem N-Atom, das sie substituieren ein bicyclisches tertiäres Amin, wie DBU (Diazabicycloundecan) oder das Pyridinsystem bilden.

Geeignete Amine für das erfindungsgemäße Verfahren sind beispielsweise Triethylamin, Tripropylamin, Tributylamin, Octyl-dimethylamin, N,N-Dimethyl-cyclohexylamin, N-Methyl-piperidin, N-Methyl-morpholin, N,N-Dimethylanilin, Pyridin, substituierte Pyridine.

Bevorzugte Amine für das erfindungsgemäße Verfahren sind solche der Formel

N(R¹¹, R¹, R¹³) (VI),

in der
- R¹¹, R¹ und R¹³: unabhängig voneinander geradkettiges oder verzweigtes C₁-C₁₂-Alkyl, Cyclopropyl, Cyclopentyl, Cyclohexyl oder Benzyl bedeuten, wobei weiterhin 2 der Reste R¹¹, R¹ und R¹³ gemeinsam mit dem N-Atom, das sie substituieren, einen 5- bis 7-gliedrigen, gesättigten heterocyclischen Ring bilden können, der ein oder zwei weitere Heteroatome aus der Gruppe N, O und S enthalten kann, wobei weiterhin einer der Substituenten R¹¹, R¹ und R¹³ Wasserstoff oder Phenyl bedeuten und wobei weiterhin alle drei Reste R¹, R und R³ gemeinsam mit dem N-Atom, das sie substituieren, ein bicyclisches tertiäres Amin oder das Pyridinsystem bilden können.

Besonders bevorzugte Amine für das erfindungsgemäße Verfahren sind solche der Formel

N(R¹, R, R³) (VII),

in der
- R¹, R und R³: unabhängig voneinander geradkettiges oder verzweigtes C₁-C₁₂-Alkyl bedeuten, wobei weiterhin einer der Reste R¹, R und R³ Cyclohexyl, Phenyl, Benzyl bedeuten kann und wobei weiterhin alle drei Reste R¹, R und R³ gemeinsam mit dem N-Atom, das sie substituieren, ein bicyclisches tertiäres Amin oder das Pyridinsystem bilden können.

Die Umsetzung des Vinylaromaten erfolgt mit schwefliger Säure H₂SO₃, oder einem ihrer wasserlöslichen Salze oder ihrem Anhydrid SO₂; der Einsatz von SO₂ ist hierbei bevorzugt.

Als molare Verhältnisse kommen in Frage: 1 bis 5 Mol, bevorzugt 1,3 bis 3,5 Mol, besonders bevorzugt 1,6 bis 2,5 Mol des oben beschriebenen Amins pro Mol Vinylaromat. Als molares Verhältnis kommt weiterhin in Frage: 1 bis 5 Mol, bevorzugt 1,3 bis 3,5 Mol, besonders bevorzugt 1,6 bis 2,5 Mol schweflige Säure oder ihre löslichen Salze oder SO₂ pro Mol Vinylaromat. In weiterhin bevorzugter Weise werden Amin und schweflige Säure bzw. ihre löslichen Salze bzw. SO₂ zueinander in äquimolarem Verhältnis eingesetzt.

Das erfindungsgemäße Verfahren läßt sich am Beispiel des Umsatzes von 4-Vinyl-pyridin mit SO₂ in Gegenwart von Triethylamin in einem wasserhaltigen Medium formelmäßig wie folgt ausdrücken:

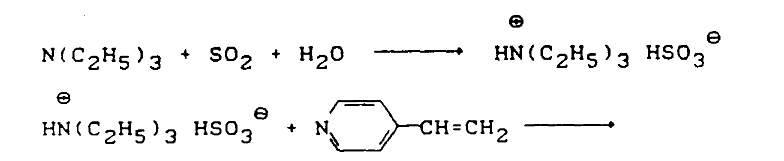

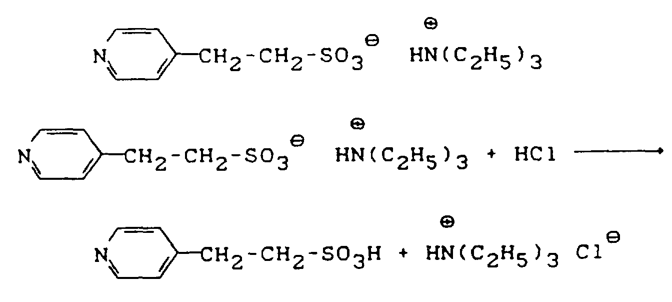

Das erfindungsgemäße Verfahren wird bei einer Temperatur von -5°C bis +65°C, bevorzugt bei 10 bis 65°C, besonders bevorzugt bei 15 bis 40°C durchgeführt. Es ist nicht erforderlich, einen überatmosphärischen oder unteratmosphärischen Druck anzulegen. Daher wird bevorzugt in der Nähe von Normaldruck, beispielsweise in einem Bereich von 0,5 bis 5 bar, besonders bevorzugt bei atmosphärischem Druck gearbeitet.

Die bevorzugte Durchführung des erfindungsgemäßen Verfahrens in der Nähe von Raumtemperatur und bei atmosphärischem Druck ergibt nur einen geringen Energiebedarf und läßt die Benutzung einer einfachen Apparatur zu.

Das Reaktionsmedium für das erfindungsgemäße Verfahren ist bevorzugt Wasser, da es keine weiteren Erfordernisse für die Rückgewinnung und Recyclisierung von organischen Lösungsmitteln mit sich bringt. Es ist jedoch gleichwohl möglich, in einem wäßrig-organischen Medium zu arbeiten, wenn mindestens eine zur schwefligen Säure äquimolare Menge an Wasser vorliegt. Mitverwendbare organische Lösungsmittel sind C₁-C₈-Alkohole, wie Methanol, Ethanol, Propanol, Isopropanol, Butanol, Isobutanol, Amylalkohol, Hexanol oder Octanol; Ketone, wie Aceton, Methyl-ethyl-keton, Methyl-isobutylketon; Nitrile, wie Acetonitril oder cyclische Ether, wie Dioxan oder Tetrahydrofuran; es ist auch möglich, im Gemisch mit wasserlöslichen organischen Stoffen der genannten Art auch mit Wasser nur schwer mischbare organische Lösungsmittel, wie Dialkylether, Toluol, Cyclohexan oder halogenierte Kohlenwasserstoffe einzusetzen. Neben der bevorzugten Variante von Wasser als Reaktionsmedium hat auch die Mitverwendung solcher organischer Lösungsmittel der genannten Art eine Bedeutung, die bei der Aufarbeitung des ausreagierten Reaktionsgemisches zur Fällung der entstandenen Aryl-ethan-sulfonsäure dienen.

Zur Durchführung des erfindungsgemäßen Verfahrens können beispielsweise das Amin und Wasser vorgelegt werden und SO₂ in diese Vorlage eingeleitet werden. In eine solche vorbereitete Lösung, in der sich ein Ammoniumhydrogensulfat gebildet hat, wird der Vinylaromat eindosiert. Die schwach exotherme Reaktion wird durch geeignete Kühlung oder geringes zusätzliches Erwärmen im oben bezeichneten Temperaturbereich gehalten. Sofern nicht bereits als Reaktionsmedium neben dem Wasser eines der oben bezeichneten organischen Lösungsmittel mitbenutzt worden war, wird nunmehr durch Zugabe eines solchen organischen Lösungsmittels die Fällung der 2-Aryl-ethan-sulfonsäuren oder ihres Salzes eingeleitet. Für diese Ausfällung haben sich besonders die niederen Alkohole, wie Methanol, Ethanol oder Isopropanol als geeignet erwiesen. Für den bevorzugten Fall, daß die 2-Aryl-ethan-sulfonsäure in Form ihrer freien Säure gewonnen werden soll, wird der mit dem organischen Fällungsmittel versetzte Reaktionsansatz mit einer starken Säure, die zur Bildung wasserlöslicher Salze führt, wie Schwefelsäure, Salpetersäure, Salzsäure oder Bromwasserstoffsäure, bevorzugt mit Salzsäure, versetzt. Gleichfalls geeignet sind starke organische Säuren, wie Sulfonsäuren, Halogencarbonsäuren und andere dem Fachmann bekannten. Wegen der einfachen Handhabung ist Salzsäure, beispielsweise als HCl-Gas, bevorzugt. Die Ausfällung wird in vorteilhafter Weise durch Abkühlen begünstigt. Die ausgefällte freie Säure wird abfiltriert und mit dem Fällungsmittel gewaschen.

Aus den Filtraten und Waschwässern kann durch Destillation das verwendete Fällungsmittel wiedergewonnen und recyclisiert werden. Der Destillationsrückstand kann sodann mit (Erd)alkalihydroxid auf freies Amin aufgearbeitet werden, welches sodann destillativ oder durch Phasentrennung gewonnen und recyclisiert wird; bei festen Aminen kommt auch eine Filtration oder Extraktion in Betracht. Vor destillativen Operationen wird in bevorzugter Weise restliches SO₂ durch Oxidationsmittel, beispielsweise Wasserstoffperoxid oxidiert. Bei höheren Aminen kann es ausreichend sein, eine gebildete organische Phase mit Wasser und dann mit verdünnter (Erd)alkalilauge auszurühren, ohne das SO₂ zu oxidieren. Auf diese Weise wird das Amin ausschließlich durch Phasentrennungen wiedergewonnen.

### Beispiel

In einem 4 l Vierhals-Glas-Kolben wurden 1390 ml = 1012 g Triethylamin vorgelegt und 815 ml Wasser zugegeben. Bei 15 bis 25°C wurden 640 g Schwefeldioxid eingeleitet, dann 30 min. nachgerührt. In diese Lösung wurden bei 25°C 532 g 4-Vinylpyridin (Gehalt ca. 98,0 %) nach Maßgabe der Wärmeabfuhr zudosiert. Zu dem Ansatz wurden anschließend 2970 g = 3760 ml Methanol zugegeben, dann wurden 550 g Salzsäuregas eingeleitet. Nach Abkühlen auf 0°C wurde die ausgefallene 2-[4]-Pyridyl-ethan-sulfonsäure filtriert und mit 1500 ml Methanol in Portionen nachgewaschen. Man erhielt ca. 1440 g feucht = 920 g trocken, Gehalt 96,5 % = 880 g 100 % (95,6 % der theoretischen Ausbeute). Die vereinigten Wäschen und Mutterlaugen wurden mit ca. 250 g 30 %igem Wasserstoffperoxid zur Vernichtung des Schwefeldioxides behandelt, dann das Methanol destillativ zurückgewonnen. Nach Zugabe von ca. 500 g 50 %iger Natronlauge wurde das Amin abdestilliert bzw. durch Phasentrennung wiedergewonnen.

## Patentansprüche

1. Verfahren zur Herstellung von 2-Aryl-ethan-sulfonsäuren und ihren Salzen, die in Form der freien Säuren der Formel
Ar¹-CH₂-CH₂-SO₃H (I)
entsprechen, in der
Ar¹ einen carbocyclischen oder heterocyclischen aromatischen Rest, der mit anellierten Benzolkernen verbunden sein kann, bedeutet,
durch Umsetzung von Vinylaromaten der Formel
Ar¹-CH=CH₂ (II),
in der
Ar¹ die obige Bedeutung hat,
mit schwefliger Säure oder ihren löslichen Salzen oder ihrem Anhydrid in einem Wasser enthaltenden Medium, dadurch gekennzeichnet, daß die Umsetzung bei einer Temperatur von -5°C bis +65°C und einem Druck von 0,5 bis 5 bar in Gegenwart eines Amins der Formel
N(R¹,R,R³) (III)
durchgeführt wird, in der
R¹, R und R³ unabhängig voneinander geradkettiges oder verzweigtes C₁-C₁₂-Alkyl, C₃-C₈-Cycloalkyl, Phenyl oder substituiertes Phenyl oder C₇-C₁₀-Aralkyl bedeuten, wobei weiterhin 2 der Reste R¹, R und R³ gemeinsam mit dem N-Atom, das sie substituieren, einen 5- bis 7-gliedrigen, gesättigten heterocyclischen Ring bilden können, der ein oder zwei weitere Heteroatome aus der Gruppe N, O und S enthalten kann, wobei weiterhin bis zu zwei der Substituenten R¹, R und R³ Wasserstoff bedeuten können und wobei weiterhin alle drei Reste R¹, R und R³ gemeinsam mit dem N-Atom, das sie substituieren, ein bicyclisches tertiäres Amin oder das Pyridinsystem bilden können.

2. Verfahren nach Anspruch 1, dadurch gekennzeichnet, daß Vinylaromaten der Formel
Ar-CH=CH₂ (IV)
umgesetzt werden, in der
Ar einen heterocyclischen aromatischen Rest bedeutet, der mit einem anellierten Benzolkern verbunden sein kann,
daß bevorzugt Vinylaromaten der Formel
Ar³-CH=CH₂ (V)
umgesetzt werden, in der
Ar³ einen Pyridinrest bedeutet, der mit einem anellierten Benzolkern verbunden sein kann.

3. Verfahren nach Anspruch 1, dadurch gekennzeichnet, daß in Gegenwart eines Amins der Formel
N(R¹¹, R¹, R¹³) (VI)
gearbeitet wird, in der
R¹¹, R¹ und R¹³ unabhängig voneinander geradkettiges oder verzweigtes C₁-C₁₂-Alkyl, Cyclopropyl, Cyclopentyl, Cyclohexyl oder Benzyl bedeuten, wobei weiterhin 2 der Reste R¹¹, R¹ und R¹³ gemeinsam mit dem N-Atom, das sie substituieren, einen 5- bis 7-gliedrigen, gesättigten heterocyclischen Ring bilden können, der ein oder zwei weitere Heteroatome aus der Gruppe N, O und S enthalten kann, wobei weiterhin einer der Substituenten R¹¹, R¹ und R¹³ Wasserstoff oder Phenyl bedeuten und wobei weiterhin alle drei Reste R¹, R und R³ gemeinsam mit dem N-Atom, das sie substituieren, ein bicyclisches tertiäres Amin oder das Pyridinsystem bilden können.

4. Verfahren nach Anspruch 2, dadurch gekennzeichnet, daß in Gegenwart eines Amins der Formel
N(R¹, R, R³) (VII)
gearbeitet wird, in der
R¹, R und R³ unabhängig voneinander geradkettiges oder verzweigtes C₁-C₁₂-Alkyl bedeuten, wobei weiterhin einer der Reste R¹, R und R³ Cyclohexyl, Phenyl oder Benzyl bedeuten kann und wobei weiterhin alle drei Reste R¹, R und R³ gemeinsam mit dem N-Atom, das sie substituieren, ein bicyclisches tertiäres Amin oder das Pyridinsystem bilden können.

5. Verfahren nach Anspruch 1, dadurch gekennzeichnet, daß bei einer Temperatur von 10 bis 65°C, bevorzugt von 15 bis 40°C gearbeitet wird.

6. Verfahren nach Anspruch 1, dadurch gekennzeichnet, daß bei atmosphärischem Druck gearbeitet wird,

7. Verfahren nach Anspruch 1, dadurch gekennzeichnet, daß 1 bis 5 Mol, bevorzugt 1,3 bis 3,5 Mol, besonders bevorzugt 1,6 bis 2,5 Mol Amin pro Mol Vinylaromat eingesetzt werden.

8. Verfahren nach Anspruch 1, dadurch gekennzeichnet, daß 1 bis 5 Mol, bevorzugt 1,3 bis 3,5 Mol, besonders bevorzugt 1,6 bis 2,5 Mol schweflige Säure oder ihre löslichen Salze oder ihr Anhydrid, bevorzugt ihr Anhydrid, pro Mol Vinylaromat eingesetzt werden.

9. Verfahren nach Anspruch 1, dadurch gekennzeichnet, daß zur Gewinnung der freien Sulfonsäuren durch Ausfällung nach Beendigung der Umsetzung eine starke Mineralsäure, die zur Bildung wasserlöslicher Salze führt, bevorzugt Salzsäure, zugesetzt wird, wobei die Ausfällung durch die Gegenwart eines mit Wasser mischbaren organischen Lösungsmittels, bevorzugt eines C₁-C₄-Alkanols, begünstigt werden kann.

10. Verfahren nach Anspruch 9, dadurch gekennzeichnet, daß nach Ausfällung und Filtration der freien Sulfonsäuren die Filtrate und Waschflüssigkeiten mit (Erd)Alkalihydroxid versetzt werden und die so in Freiheit gesetzten Amine destillativ oder durch Phasentrennung gewonnen und recyclisiert werden.

## Claims

1. Process for the preparation of 2-aryl-ethane-sulphonic acids and salts thereof, which, in the form of the free acids, correspond to the formula
Ar¹-CH₂-CH₂-SO₃H (I)
in which
Ar¹ denotes a carbocyclic or heterocyclic aromatic radical which can be bound to anellated benzene nuclei,
by reaction of vinyl aromatic compounds of the formula
Ar¹-CH=CH₂ (II),
in which
Ar¹ has the above definition,
with sulphurous acid or soluble salts thereof or the anhydride thereof in a water-containing medium, characterised in that the reaction is carried out at a temperature of -5°C to +65°C and at a pressure of 0.5 to 5 bar in the presence of an amine of the formula
N(R¹,R,R³) (III)
in which
R¹, R and R³, independently of each other, denote straight-chain or branched C₁-C₁₂-alkyl, C₃-C₈-cycloalkyl, phenyl or substituted phenyl or C₇-C₁₀-aralkyl, where, furthermore, 2 of the radicals R¹, R and R³ together with the N atom which they substitute can form a 5- to 7-member saturated heterocyclic ring which can contain one or two further hetero atoms selected from the group comprising N, O and S, where, furthermore, up to two of the substituents R¹, R and R³ can denote hydrogen and where, furthermore, all three radicals R¹, R and R³ together with the N atom which they substitute can form a bicyclic tertiary amine or the pyridine system.

2. Process according to Claim 1, characterised in that vinyl aromatic compounds of the formula
Ar-CH=CH₂ (IV)
are reacted, in which
Ar denotes a heterocyclic aromatic radical which can be bound to an anellated benzene nucleus,
in that vinyl aromatic compounds of the formula
Ar³-CH=CH₂ (V)
are preferably reacted, in which
Ar³ denotes a pyridine radical which can be bound to an anellated benzene nucleus.

3. Process according to Claim 1, characterised in that the reaction is carried out in the presence of an amine of the formula
N(R¹¹,R¹,R¹³) (VI)
in which
R¹¹, R¹ and R¹³, independently of each other, denote straight-chain or branched C₁-C₁₂-alkyl, cyclopropyl, cyclopentyl, cyclohexyl or benzyl, where, furthermore, 2 of the radicals R¹¹, R¹ and R¹³, together with the N atom which they substitute, can form a 5- to 7-member saturated heterocyclic ring which can contain one or two further hetero atoms selected from the group comprising N, O and S, where, furthermore, one of the substituents R¹¹, R¹ and R¹³ denotes hydrogen or phenyl and where, furthermore, all three radicals R¹, R and R³, together with the N atom which they substitute, can form a bicyclic tertiary amine or pyridine system.

4. Process according to Claim 2, characterised in that the reaction is carried out in the presence of an amine of the formula
N(R¹,R,R³) (VII)
in which
R¹, R and R³, independently of each other, denote straight-chain or branched C₁-C₁₂-alkyl, where, furthermore, one of the radicals R¹, R and R³ can denote cyclohexyl, phenyl or benzyl and where, furthermore, all three radicals R¹, R and R³, together with the N atom which they substitute, can form a bicyclic tertiary amine or the pyridine system.

5. Process according to Claim 1, characterised in that a temperature from 10° to 65° C, preferably 15° to 40° C, is employed.

6. Process according to Claim 1, characterised in that atmospheric pressure is employed.

7. Process according to Claim 1, characterised in that 1 to 5 mol, preferably 1.3 to 3.5 mol, particularly preferably 1.6 to 2.5 mol, of amine are used per mole of vinyl aromatic compound.

8. Process according to Claim 1, characterised in that 1 to 5 mol, preferably 1.3 to 3.5 mol, particularly preferably 1.6 to 2.5 mol, of sulphurous acid or its soluble salts or its anhydride, preferably its anhydride, are used per mole of vinyl aromatic compound.

9. Process according to Claim 1, characterised in that, in order to obtain the free sulphonic acids by precipitation after completion of the reaction, a strong mineral acid which leads to the formation of water-soluble salts, preferably hydrochloric acid, is added, where the precipitation can be promoted by the presence of a water-miscible organic solvent, preferably a C₁-C₄-alkanol.

10. Process according to Claim 9, characterised in that after precipitation and filtration of the free sulphonic acids, alkali (alkaline earth) metal hydroxide is added to the filtrates and washing liquids and the amines thus liberated are recovered by distillation or phase separation and are recycled.

## Revendications

1. Procédé de préparation des acides 2-aryléthanesulfoniques et de leurs sels répondant, à l'état d'acide libre, à la formule
Ar¹-CH₂-CH₂-SO₃H (I)
dans laquelle
Ar¹ représente un radical aromatique homogène ou hétérogène qui peut être condensé avec des noyaux benzéniques,
par réaction de composés vinylaromatiques de formule
Ar¹-CH=CH₂ (II),
dans laquelle
Ar¹ a les significations indiquées ci-dessus,
avec l'acide sulfureux ou ses sels solubles ou son anhydride dans un milieu contenant de l'eau, le procédé se caractérisant en ce que la réaction est réalisée à une température comprise entre -5 et +65°C et sous une pression de 0,5 à 5 bar en présence d'une amine de formule
N(R¹, R, R³) (III)
dans laquelle
R¹, R et R³ représentent chacun, indépendamment les uns des autres, un groupe alkyle à chaîne droite ou ramifiée en C₁-C₁₂, cycloalkyle en C₃-C₈, phényle ou phényle substitué ou aralkyle en C₇-C₁₀ ; deux des symboles R¹, R et R³ pouvant également représenter ensemble et avec l'atome d'azote dont ils sont substituants un noyau hétérocyclique saturé de 5 à 7 chaînons qui peut contenir un ou deux autres hétéroatomes choisis parmi N, O et S ; jusqu'à deux des symboles R¹, R et R³ pouvant représenter l'hydrogène ; et les trois symboles R¹, R et R³ pouvant encore représenter ensemble et avec l'atome d'azote dont ils sont substituants, une amine tertiaire bicyclique ou le système pyridique.

2. Procédé selon la revendication 1, caractérisé en ce que l'on convertit des composés vinylaromatiques de formule
Ar-CH=CH₂ (IV)
dans laquelle Ar représente un radical aromatique hétérocyclique qui peut être condensé avec un noyau benzénique,
et de préférence des composés vinylaromatiques de formule
Ar³-CH=CH₂ (V)
dans laquelle Ar³ représente un radical de pyridine qui peut être condensé avec un noyau benzénique.

3. Procédé selon la revendication 1, caractérisé en ce que l'on opère en présence d'une amine de formule
N(R¹¹, R¹, R¹³) (VI),
dans laquelle
R¹¹, R¹ et R¹³ représentent chacun, indépendamment les uns des autres, un groupe alkyle à chaîne droite ou ramifiée en C₁-C₁₂, cyclopropyle, cyclopentyle, cyclohexyle ou benzyle ; deux des symboles R¹¹, R¹ et R¹³ pouvant également former ensemble et avec l'atome d'azote dont ils sont substituants un noyau hétérocyclique saturé contenant 5 à 7 chaînons et qui peut contenir un ou deux autres hétéroatomes choisis parmi N, O et S ; l'un des symboles R¹¹, R¹ et R¹³ pouvant représenter l'hydrogène ou un groupe phényle ; et les trois symboles R¹, R et R³ pouvant former ensemble et avec l'atome d'azote dont ils sont substituants une amine tertiaire bicyclique ou le système pyridique.

4. Procédé selon la revendication 2, caractérisé en ce que l'on opère en présence d'une amine de formule
N(R¹, R, R³) (VII),
dans laquelle
R¹, R et R³ représentent chacun, indépendamment les uns des autres, un groupe alkyle à chaîne droite ou ramifiée en C₁-C₁₂, l'un des symboles R¹, R et R³ pouvant également représenter un groupe cyclohexyle, phényle, benzyle ; et d'autre part les trois symboles R¹, R et R³ pouvant représenter ensemble et avec l'atome d'azote dont ils sont substituants une amine tertiaire bicyclique ou le système pyridique.

5. Procédé selon la revendication 1, caractérisé en ce que l'on opère à une température de 10 à 65°C, de préférence de 15 à 40°C.

6. Procédé selon la revendication 1, caractérisé en ce que l'on opère à pression atmosphérique.

7. Procédé selon la revendication 1, caractérisé en ce que l'on met en oeuvre de 1 à 5 mol, de préférence de 1,3 à 3,5 mol et dans les meilleures conditions de 1,6 à 2,5 mol de l'amine par mole du composé vinylaromatique.

8. Procédé selon la revendication 1, caractérisé en ce que l'on met en oeuvre de 1 à 5 mol, de préférence de 1,3 à 3,5 mol et dans les meilleures conditions de 1,6 à 2,5 mol d'acide sulfureux ou de l'un de ses sels solubles ou de son anhydride, de préférence de ce demier, par mole du composé vinylaromatique.

9. Procédé selon la revendication 1, caractérisé en ce que, pour isoler les acides sulfoniques libres par précipitation après la réaction, on ajoute un acide minéral fort provoquant la formation de sels solubles dans l'eau, de préférence l'acide chlorhydrique, la précipitation pouvant être avantagée par la présence d'un solvant organique miscible à l'eau, de préférence un alcanol en C₁-C₄.

10. Procédé selon la revendication 9, caractérisé en ce que, après précipitation et filtration des acides sulfoniques libres, on ajoute aux filtrats et aux eaux de lavage un hydroxyde alcalin ou alcalino-terreux, ce qui provoque la libération de l'amine qu'on récupère par distillation ou par séparation de phases et qu'on recycle.
